(19) Europäisches Patentamt
European Patent Office
Office européen des brevets

(11) EP 0 894 813 B1

(12) **FASCICULE DE BREVET EUROPEEN**

(45) Date de publication et mention de la délivrance du brevet:
**10.11.2004 Bulletin 2004/46**

(51) Int Cl.[7]: **C08G 18/38**, C08G 18/28, C08G 18/66, A61L 33/00, B01D 63/02

(21) Numéro de dépôt: **98420135.0**

(22) Date de dépôt: **29.07.1998**

(54) **Articles médicaux non cytotoxiques en polyuréthane**

Nicht zytotoxische medizinische Artikel aus Polyurethanen

Non cytotoxic medical polyurethane articles

(84) Etats contractants désignés:
**AT BE CH DE ES FR GB IE IT LI NL SE**

(30) Priorité: **31.07.1997 FR 9710037**

(43) Date de publication de la demande:
**03.02.1999 Bulletin 1999/05**

(73) Titulaire: **HOSPAL INDUSTRIE**
**F-69883 Meyzieu Cédex (FR)**

(72) Inventeurs:
- **Esposito, Guy**
**01700 Beynost (FR)**
- **Herze, Pierre-Yves**
**1050 Ixelles (BE)**

(74) Mandataire: **Sutto, Luca et al**
**Gambro Intellectual Property Department,**
**P.O. Box 98**
**41037 Mirandola (MO) (IT)**

(56) Documents cités:
**EP-A- 0 383 596**
**FR-A- 2 147 154**
**US-A- 3 354 241**
**US-A- 3 483 147**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

EP 0 894 813 B1

Printed by Jouve, 75001 PARIS (FR)

## Description

L'invention a pour objet des articles médicaux non cytotoxiques après stérilisation ou désinfection par un procédé oxydant, à base de compositions génératrices de polyuréthane, et plus particulièrement des masses d'empotage pour échangeurs médicaux à membranes planes ou à fibres creuses. L'invention a également pour objet un procédé de préparation de ces articles médicaux en polyuréthane permettant de limiter fortement la cytotoxicité résultant de la stérilisation ou de la désinfection par un procédé oxydant tel que les rayonnements ionisants (irradiation gamma, faisceau d'électrons), les peroxydes gazeux (stérilisation dite par plasma froid), les peroxydes liquides ou tout autre procédé physique ou chimique faisant intervenir une réaction d'oxydation susceptible de dénaturer le matériau stérilisé.

Selon le cas, les masses d'empotage en polyuréthane sont destinées à former :

. une cloison cylindrique de séparation des deux compartiments d'un échangeur médical dont la membrane est constituée par un faisceau de fibres creuses semi-perméables. L'opération consistant à réaliser une telle cloison de séparation est usuellement désignée sous le vocable "d'empotage" ;

. ou un joint étanche dans un échangeur médical comprenant une membrane plane semi-perméable. L'opération consistant à réaliser un tel joint est usuellement désignée sous le vocable de "garnissage d'étanchéité". Néanmoins, afin de simplifier la présente description, cette opération sera également désignée sous le vocable "d'empotage".

Dans un souci de clarté de la présente description, le vocable de "joint" sera utilisé pour désigner indifféremment un joint étanche dans des échangeurs médicaux à membranes planes semi-perméables ou une cloison cylindrique de séparation dans des échangeurs médicaux dont la membrane est constituée par un faisceau de fibres creuses semi-perméables.

La présente invention est en particulier utile pour la fabrication des échangeurs pour des applications médicales sous la forme, par exemple, de dialyseurs, hémofiltres et oxygénateurs.

Il est de pratique courante de fabriquer des échangeurs pour des applications médicales en suivant les étapes générales qui suivent :

- préparer une membrane semi-perméable à partir d'une membrane plane ou conformer un faisceau de fibres creuses semi-perméables à partir de fibres creuses ;
- monter la membrane semi-perméable ou bien le faisceau de fibres creuses dans un boîtier et former, selon le cas, un joint étanche ou une cloison cylindrique de séparation des deux compartiments, à l'aide d'une composition adhésive génératrice de polyuréthane ;
- le cas échéant, fixer des embouts au boîtier, et
- stériliser l'appareil médical obtenu.

Les compositions adhésives génératrices de polyuréthane utilisées pour préparer un joint dans un échangeur médical, comprennent, d'une manière générale, avant polymérisation, un ou plusieurs polyisocyanates, un ou plusieurs polyols et, éventuellement, un ou plusieurs agents réticulants polyfonctionnels et/ou un ou plusieurs catalyseurs.

Le polyuréthane, une fois qu'il est durci, a pour fonction essentielle de former un joint étanche afin qu'il n'y ait pas d'infiltration entre les deux compartiments des échangeurs ou avec l'extérieur. Le risque d'infiltration doit, en particulier, être évité entre le compartiment à sang et le compartiment à dialysat des échangeurs médicaux pour le traitement du sang. Pour ce faire, la composition adhésive de polyuréthane doit présenter une adhérence satisfaisante avec les membranes semi-perméables des échangeurs, quelle que soit la nature chimique des matières qui les constituent. Egalement, cette composition doit présenter une adhérence satisfaisante avec les éléments des échangeurs avec lesquels elle est mise en contact tels que le boîtier.

Une autre qualité importante exigée des échangeurs à usage biomédical est la biocompatibilité des masses d'empotage en polyuréthane, durcies et stérilisées, plus spécialement leur non cytotoxicité. Or, l'étape de stérilisation ou de désinfection par un procédé oxydant, surtout lorsqu'il s'agit d'une stérilisation par irradiation, peut rendre le polyuréthane cytotoxique.

Antérieurement, afin de former des masses d'empotage non cytotoxiques, diverses solutions ont été proposées, ainsi :

- dans le brevet américain US-4,332,927 il a été proposé des compositions génératrices de polyuréthane comprenant au moins un prépolymère à terminaisons isocyanato(-NCO), au moins un polyol et une quantité catalytique d'un composé d'étain dialcoyle dicarboxylé ;
- dans les brevets européens n°0393545 et n°0413265 et le brevet américain US-5,306,798, diverses compositions

adhésives génératrices de polyuréthane à base de diphényl méthane diisocyanates (MDI) ou de dérivé de MDI, et de polyols spécifiques ont été proposées.

D'une manière différente et surprenante, la demanderesse a trouvé qu'il est possible de fabriquer des articles médicaux en polyuréthane stérilisés ou désinfectés par un procédé oxydant, qui sont non cytotoxiques, en particulier des masses d'empotage en polyuréthane, qui sont en outre suffisamment adhérentes avec les membranes semi-perméables des échangeurs mais non cytotoxiques après stérilisation ou désinfection par un procédé oxydant. Conformément à l'invention, on part d'une composition génératrice de polyuréthane contenant :

- au moins un polyisocyanate, de préférence non aromatique, à l'état de monomère ou de prépolymère ;
- au moins un polyol, à l'état de monomère ou de prépolymère ;
- au moins un composé organique contenant une ou plusieurs fonctions phosphites, la quantité de ce composé organique étant au moins égale à 1% en poids rapporté au poids total du ou des polyols, ce composé organique étant en outre non aromatique et porteur d'au moins un groupement hydroxy(-OH), libre et capable de réagir avec un groupement isocyanato(-NCO) ; et
- avantageusement, la composition génératrice de polyuréthane comprend en outre au moins un catalyseur de la réaction de polymérisation d'un polyisocyanate et d'un polyol.

La présente invention a également pour objet un procédé permettant de réduire la cytotoxicité des articles médicaux en polyuréthane, en particulier des masses d'empotage en polyuréthane, susceptible d'apparaître après stérilisation ou désinfection par un procédé oxydant, caractérisé en ce que l'on prépare une composition génératrice de polyuréthane à partir de :

- au moins un polyisocyanate, de préférence non aromatique, à l'état de monomère ou de prépolymère ;
- au moins un polyol ;
- au moins un composé organique contenant une ou plusieurs fonctions phosphites en une quantité d'au moins 1 % en poids rapporté au poids total du ou des polyols, ce composé organique étant en outre non aromatique et porteur d'au moins un groupement hydroxy(-OH) libre et capable de réagir avec un groupement isocyanato(-NCO) ; et
- le cas échéant, au moins un catalyseur de la réaction de polymérisation d'un polyisocyanate et d'un polyol.

Ainsi, le composé organique contenant une ou plusieurs fonctions phosphites peut être lié par liaison chimique dans le réseau polyuréthane.

Dans le cadre de la présente invention, par articles médicaux en polyuréthane non cytotoxiques, on entend des articles conduisant à un pourcentage d'inhibition de croissance cellulaire (% ICG) fortement réduit grâce à la présence d'au moins un composé organique contenant une ou plusieurs fonctions phosphites. Ce pourcentage d'ICG est de préférence au plus égal à 30% en moyenne sur au moins 3 échantillons, quand les articles médicaux en polyuréthane sont soumis aux essais biologiques des matériaux et dispositifs médicaux et dentaires, partie 5 : méthodes in vitro, de la norme ISO 10-993, complétée par les conditions de mesure de la cytotoxicité utilisées par la demanderesse. Ces conditions spécifiques de mesure de la cytotoxicité sont énoncées ci-après, avec les exemples.

Une caractéristique essentielle de l'invention réside dans l'utilisation d'au moins un composé organique contenant une ou plusieurs fonctions phosphites pour préparer des articles médicaux en polyuréthane non cytotoxiques et plus spécialement des masses d'empotage pour échangeurs médicaux à membranes planes ou à fibres creuses.

La formule générale (I) des composés organiques convenant à l'invention, qui comprend au moins une fonction phosphite est de préférence la suivante :

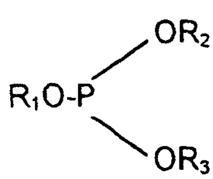

dans laquelle :

- $R_1$, $R_2$ et $R_3$ sont identiques ou différents et représentent un radical alkyle, $R_1$, $R_2$ et $R_3$ peuvent être linéaires ou ramifiés, acycliques ou cycliques ; $R_1$, $R_2$ et $R_3$ sont exempts de groupements aromatiques ;

- au moins un radical $R_1$, $R_2$ ou $R_3$ comprend au moins un groupement hydroxy(-OH) ;

La formule générale (I) précitée fait apparaître une fonction phosphite.

Avantageusement, au moins un radical $R_1$, $R_2$ ou $R_3$ comprend une ou plusieurs autres fonctions phosphites selon la formule générale (I).

Dans la suite de la description, par souci de simplification, les composés organiques (I) seront appelés "phosphites (I)".

Grâce à la présence d'au moins un groupement hydroxy, les phosphites (I) sont avantageusement hydrophiles. De préférence, le ou les groupements hydroxy des phosphites (I) sont primaires ou secondaires.

Avantageusement, on choisit des phosphites (I) dont le point de fusion est inférieur ou égal à 40°C et, encore mieux, qui sont liquides à température ambiante, c'est-à-dire à une température de l'ordre de 20-25°C.

De préférence, on utilise des phosphites (I) contenant au moins deux fonctions phosphites, encore mieux, trois fonctions phosphites.

Les radicaux alkyles $R_1$, $R_2$ ou $R_3$ des phosphites (I) peuvent aussi comporter un ou plusieurs hétéroatomes, de préférence un ou plusieurs atomes d'oxygène pour former une ou plusieurs fonctions éthers. Ainsi, les phosphites (I) peuvent comporter un ou plusieurs groupements, monovalents ou divalents, choisis parmi le diéthylèneglycol, le triéthylèneglycol, le dipropylèneglycol et le dibutylèneglycol.

A titre d'exemples de phosphites (I) convenant à l'invention, on peut citer l'heptakis(dipropylèneglycol)triphosphite (nom abrégé PTP) et le tris(dipropylèneglycol)phosphite. Le composé phosphite (I) préféré est le PTP.

La quantité de phosphite (I) à prévoir pour atteindre des résultats satisfaisants au test de cytotoxicité (i.e. de préférence au plus 30% d'inhibition de croissance cellulaire, en moyenne sur au moins 3 échantillons) est au moins égale à 1 % en poids rapporté au poids total du ou des polyols. Il est préférable que la quantité de phosphites (I) n'excède pas 10 % en poids (rapporté au poids total des polyols), afin de limiter une compétition avec le polyol réticulant. De préférence, la quantité de phosphites (I) est comprise entre 1,5 % et 8 % en poids rapporté au poids total du ou des polyols.

A titre d'exemples de polyols susceptibles de convenir à l'invention, on peut utiliser : l'huile de ricin ; les esters de polyol et de l'acide ricinoléique ; des polyéther polyols tels que le polyoxypropylène glycol et des polytétraméthylène éther glycols ; des homopolymères ou copolymères de butadiène porteurs d'au moins deux groupements hydroxy ; des esters de polyol et d'acide gras tel que l'huile de soja ou de l'huile de ricin, ou des esters de diacide saturé ou non, et d'éthylène glycol comme l'adipate de polyéthylène glycol ; la N,N,N',N'-tétrakis (hydroxy-propyl) éthylènediamine ; des polycaprolactone polyols ; des oligomères polyols, en particulier des dimères polyols pouvant comprendre un groupement hydrocarboné, cyclique et saturé, et formés par condensation et réduction complète de deux acides gras insaturés, ou formés par condensation, réduction partielle et estérification de deux acides gras insaturés ; des prépolymères polyols obtenus par réaction d'un excès de polyols avec un polyisocyanate, de préférence un polyisocyanate non aromatique ; des mélanges de deux ou plus des polyols précités.

A titre d'exemple de mélanges de polyols convenant à l'invention, on peut citer :

- un premier mélange comprenant :
  - . environ 45 % en poids d'un polyol à base d'esters de polyol et de polyéther polyols tel que le produit commercialisé par HENKEL sous le nom SOVERMOL 1080 ;
  - . environ 25 % en poids d'huile de ricin ;
  - . environ 30 % en poids de polycaprolactone polyol tel qu'un mélange 50/50 des polyols commercialisés par SOLVAY sous les noms CAPA 305 et CAPA 301.
- un second mélange comprenant :
  - . environ 70 % en poids d'un ester d'un dimère diacide et d'éthylène glycol tel que le produit commercialisé par UNICHEMA sous le nom PRIPLAST 3193;
  - . environ 15 % d'huile de ricin ;
  - . environ 15 % de N,N,N',N'-tétrakis (2-hydroxy-propyl)éthylène diamine.

Les polyisocyanates convenant à l'invention sont de préférence non aromatiques, c'est-à-dire exempts d'un ou plusieurs noyaux benzéniques, naphtalèniques, anthracéniques, etc. A titre d'exemples de polyisocyanates non aromatiques, à l'état de monomères ou de prépolymères, susceptibles de convenir à l'invention, on peut citer les polyisocyanates aliphatiques ou cycloaliphatiques, tels que le dicyclohexylméthyl-4,4'-diisocyanate (HMDI), l'isophorone-diisocyanate (IPDI) correspondant au 3-isocyanato méthyl-3,5,5-triméthylcydohexylisocyanate, le triméthyl hexaméthylène diisocyanate, l'hexaméthylène diisocyanate (HDI) et ses dérivés de condensation tels que le biuret de HDI

et l'isocyanurate de HDI. Des polyisocyanates aromatiques peuvent également être utilisées.

La quantité de polyisocyanate mise à réagir avec le polyol devra être suffisante pour fournir au moins un groupement isocyanato par groupement hydroxy de polyol. Un rapport en nombre NCO/OH égal ou supérieur à 1, de préférence égal ou supérieur à 1,1 est avantageux.

Bien entendu, les compositions de polyuréthane selon l'invention peuvent renfermer divers additifs classiquement utilisés dans le domaine technique en cause, tels que :

- au moins un catalyseur pour accélérer la réticulation, de préférence à raison de 0,01 à 2 % en poids de la composition totale. A titre d'exemple de catalyseur convenant à la présente invention, on peut citer les carboxylates d'étain tels que le dibutyl dilaurate d'étain (DBTL). La présence d'un catalyseur est recommandée lorsque les polyisocyanates sont non aromatiques ;
- au moins un promoteur d'adhésion pour conférer au polyuréthane durci une adhérence améliorée aux fibres creuses ou membranes planes semi-perméables, en particulier quand l'échangeur médical est constitué d'une membrane semi-perméable chargée négativement [i.e. une membrane contenant des charges négatives en excès détectable, en particulier par des mesures d'écoulement (potentiel Zéta)]. De préférence, on choisit une PEI de masse moléculaire moyenne en poids allant d'environ 600 à environ 10 000, plus aisée à mettre en oeuvre du fait de leur viscosité plus faible à température ambiante. La PEI peut être mise en oeuvre selon deux procédés différents (a) ou (b) qui suivent :

    a) la PEI peut servir à traiter la surface extérieure des membranes semi-perméables. Elle est alors appliquée sur la surface extérieure des fibres ou des canaux formés par les membranes semi-perméables planes;
    b) la PEI peut être incorporée dans la composition adhésive génératrice de polyuréthane. Elle est alors mélangée avec l'un ou plusieurs des composants servants à préparer une composition adhésive génératrice de polyuréthane.

Un brevet représentatif de la technique de l'utilisation de PEI comme promoteur d'adhésion des masses d'empotage est la demande de brevet européen n°0710683.

Divers procédés de préparation des articles médicaux en polyuréthane selon l'invention peuvent être utilisés.

Conformément à un mode de réalisation préféré, on prépare une composition génératrice de polyuréthane à partir de deux composants qui sont conservés séparément, respectivement un premier composant constitué d'au moins un polyisocyanate à l'état de monomère ou de prépolymère et un second composant constitué d'au moins un polyol et d'au moins un phosphite (I), non aromatique et porteur d'au moins un groupement OH libre, à raison d'au moins 1 % en poids de phosphite (I) rapporté au poids du ou des polyols et, le cas échéant, d'au moins un catalyseur et/ou d'au moins un promoteur d'adhésion. On mélange ces deux composants jusqu'à l'obtention d'un mélange homogène au moment de la fabrication de l'article médical tel qu'un joint étanche en polyuréthane.

Egalement, la composition génératrice de polyuréthane peut être présentée, avant utilisation, en trois composants, respectivement un premier composant constitué d'au moins un polyisocyanate, à l'état de monomère ou de prépolymère, un second composant constitué d'au moins un polyol et, éventuellement, d'au moins un catalyseur et/ou au moins un promoteur d'adhésion, et un troisième composant constitué d'au moins un composé phosphite (I) non aromatique et porteur d'au moins un groupement OH libre, à raison d'au moins 1 % en poids de phosphite (I) rapporté au poids du ou des polyols.

Les compositions adhésives génératrices de polyuréthane selon l'invention sont particulièrement bien adaptées aux membranes semi-perméables chargées négativement et sont conformées dans un seul type de matériau qui comprend notamment un homo- ou copolymère de l'acrylonitrile sous forme d'une membrane plane ou d'un faisceau de fibres creuses.

Un tel matériau, quand il est constitué d'un ou de plusieurs copolymères d'acrylonitrile, peut comprendre :

(1) un copolymère de l'acrylonitrile et d'au moins un monomère anionique ou anionisable, renfermant, le cas échéant, des unités provenant d'au moins un autre monomère à insaturation oléfinique capable d'être copolymérisé avec l'acrylonitrile, ou
(2) un copolymère de l'acrylonitrile et d'au moins un monomère anionique ou anionisable et d'au moins un monomère non ionique et non ionisable.

Certains de ces composés macromoléculaires, ainsi que les divers monomères susceptibles d'être retenus comme matières premières de leur fabrication, sont plus amplement décrits dans le brevet américain n°4,545,910 redélivré sous le n°Re.34239.

Parmi ces composés macromoléculaires, ceux avec lesquels les compositions adhésives génératrices de polyuréthane selon l'invention sont particulièrement bien adaptées sont définis sous (1) ci-avant, surtout quand la

membrane semi-perméable est à l'état d'hydrogel. En particulier, l'invention convient particulièrement bien à ceux pour lesquels le comonomère anionique ou anionisable est oléfiniquement insaturé et porteur de groupements anioniques choisis parmi les groupes sulfonate, carboxyle, phosphate, phosphonate et sulfate, et plus particulièrement encore, quand ce comonomère est le méthallylsulfonate de sodium.

Bien entendu, la nature précise du contre-ion des groupements anioniques n'est pas essentielle au bon fonctionnement de l'invention.

Parmi les monomères à insaturation oléfinique capables d'être copolymérisés avec l'acrylonitrile, on peut citer les acrylates d'alkyle et, en particulier, l'acrylate de méthyle.

Les exemples ci-après illustrent l'invention sans aucunement en limiter la portée.

## EXEMPLES

Préparation de polyuréthane (PUR à partir d'une composition adhésive ), en deux composants, incorporant ou non un composé phosphite.

Dans une cuve munie d'un agitateur, on introduit une quantité déterminée de polyol. On pèse une quantité déterminée de polyéthylèneimine (PEI) de masse moléculaire moyenne de l'ordre de 600 et on introduit cette quantité dans la cuve. Le cas échéant, on pèse une quantité déterminée de phosphite (I) et on introduit cette quantité à son tour dans la cuve. On agite alors, sous atmosphère d'azote ou d'air sec jusqu'à homogénéisation. Le mélange est ensuite dégazé sous vide, à température ambiante ou à chaud (maximum 40°C), avant son utilisation pour la préparation du polyuréthane par mélange avec un polyisocyanate, lui-même dégazé sous vide (pour éviter la présence de bulles).

Le taux de catalyseur est ajusté suivant le temps de gel désiré.

Pour environ la moitié de sa composition (premier composant), le PUR est à base d'une partie dite isocyanate. L'autre partie de la composition servant à préparer le PUR (second composant) contient le mélange stable de polyol, de PEI, de catalyseur précité et le cas échéant, de phosphite (I). Les proportions de la partie isocyanate et de la partie polyol sont calculées en fonction du poids équivalent de groupement isocyanato (NCO) et du poids équivalent de groupement hydroxy (OH) afin d'avoir un ratio NCO/OH égal à 1,1.

Pour les exemples 1a, 1b, 2a et 2b, la nature chimique et la quantité des polyols, du phosphite (I), du polyisocyanate et du catalyseur figurent au tableau ci-après.

| Nature chimique des composants | Exemple 1a | Exemple 1b | Exemple 2a | Exemple 2b |
|---|---|---|---|---|
| | Quantité de chaque composant (en % en poids) | | | |
| Composant isocyanate à base d'hexaméthylène diisocyanate (HDI) | 48,7 | 48,7 | 0 | 0 |
| Composant polyol à base de (% en poids) :<br>- 45% d'un produit à base d'esters de polyol et de polyether polyols commercialisé sous le nom SOVERMOL 1080 par HENKEL (nombre de OH en mg KOH/g de produit = 170)<br>- 25% d'huile de ricin<br>- 15% de polycaprolactone polyol commercialisé sous le nom CAPA 305 par SOLVAY (poids moléculaire = 540 ; nombre de OH en mg KOH/g de produit = 310)<br>- 15% de polycaprolactone polyol commercialisé sous le nom CAPA 301 par SOLVAY (poids moléculaire = 300 ; nombre de OH en mg KOH/g de produit = 560)<br>- 0,1% (*) de PEI<br>- 0,15% (*) de dibutyl dilaurate d'étain (DBTL) | 51,3 | 51,3 | 0 | 0 |
| Composant isocyanate à base d'isocyanurate de HDI (commercialisé par BAYER sous la dénomination DESMODUR W) | 0 | 0 | 36,3 | 36 |

(suite)

| Nature chimique des composants | Exemple 1a | Exemple 1b | Exemple 2a | Exemple 2b |
|---|---|---|---|---|
| | Quantité de chaque composant (en % en poids) | | | |
| Composant polyol à base de (% en poids) :<br>- 70% d'un dimèrate d'éthylène glycol (1) de poids moléculaire égal à 1000 commercialisé par UNICHEMA sous le nom PRIPLAST 3193 (nombre de OH en mg KOH/g de produit = 106)<br>- 15 % d'huile de ricin<br>- 15% de N,N,N',N'-tétrakis(2-hydroxy-propyl) éthylène diamine<br>- 0,15% ( * ) de PEI<br>- 0,5% ( * ) de DBTL | 0 | 0 | 63,7 | 63,7 |
| Composé phosphite (I) ajouté au composant polyol Heptakis(dipropylèneglycol)triphosphite | 0 | 3( * ) | 0 | 5(*) |

(*) pourcentage en sus par rapport à la masse de polyol.

(1) Le dimérate d'éthylène glycol est obtenu en faisant réagir (estérification) un dimère diacide et de l'éthylène glycol. Le dimère diacide est issu de l'estérification d'un dimère diol en C-36 (i.e. 36 atomes de carbones) et de diacides.

Empotage des fibres creuses

L'empotage avec une composition adhésive génératrice de polyuréthane ou PUR nécessite, au préalable, un séchage, au minimum, des extrémités du faisceau de fibres qui seront en contact avec le PUR.

Ensuite, on prépare la composition adhésive génératrice de polyuréthane en mélangeant les premier et second composants mentionnés au paragraphe précédent pour les besoins des exemples.

Aussitôt après, on coule la composition dans un réservoir muni de tubulures reliées aux deux extrémités d'un boîtier tubulaire où un faisceau de fibres creuses a été introduit et où il doit être empoté à ses deux extrémités. Préalablement à cette opération, le boîtier a été muni de bouchons à ses extrémités pour contenir la colle lors de l'empotage proprement dit.

On met en rotation le boîtier contenant le faisceau de fibres autour d'un axe perpendiculaire à l'axe longitudinal du faisceau et passant à mi-longueur du dispositif. Sous l'effet de la force centrifuge, la composition est déplacée aux extrémités du faisceau de fibres et vient enrober celles-ci. La composition pénètre également à l'intérieur des fibres mais cette pénétration est limitée par la compression de l'air prisonnier à l'intérieur des fibres. En outre, cette pénétration est maîtrisée en jouant sur deux paramètres : la force centrifuge (i.e. la vitesse de rotation du dispositif) et la température de l'air.

Après polymérisation de la composition, les bouchons sont enlevés et la masse d'empotage est tranché à un niveau au-delà de la pénétration de la composition dans les fibres de façon à ce que les fibres soient ouvertes pour permettre la circulation de fluide à l'intérieur des fibres.

Une fois l'assemblage terminé, le produit obtenu est ensaché hermétiquement pour être protégé de toute contamination microbiologique après stérilisation.

Le mode de stérilisation choisi est l'irradiation gamma à une dose d'irradiation au moins égale à 25 kGy garantissant une probabilité négligeable de contamination microbiologique après stérilisation.

Protocole de mesure de la cytotoxicité d'un polyuréthane (PUR)

Dans les exemples, on a mesuré la cytotoxicité des compositions adhésives de polyuréthane (PUR) durcies conformément aux recommandations de la norme ISO 10-993, partie 5, complétées de la façon suivante par la société HOSPAL :

A J1 (1er jour), dans des conditions aseptiques, une lignée de cellules fibroblastiques de souris (L929) est ensemencée à faible densité au fond de puits de culture (≅ 5000 cellules par puits de 0,32 $cm^2$). Les cellules cultivées dans un milieu de culture additionné de 10 % de sérum de veau foetal (contenant des facteurs de croissance) adhèrent au plastique avant de se diviser.

A J2 (2ème jour), au moment où les cellules entrent en phase de croissance logarithmique, les cellules sont mises en contact avec l'éluat aqueux du PUR étudié, susceptible de contenir des substances extractibles.

Les conditions de préparation de l'éluat aqueux du PUR étudié, notamment les rapports surface/volume et

température/durée sont décrites dans la norme ISO 10-993, partie 12.

L'éluat aqueux du PUR est dilué au demi avec un milieu de culture 2x (deux fois concentré). Une dilution de l'éluat au ¼ a aussi été testée après dilution au demi dans un milieu de culture 1x de la solution précédente.

A J5 (5ème jour), les puits de culture sont vidés, le tapis cellulaire est lavé et la densité et la viabilité des cellules sont quantifiées à l'aide d'une solution standardisée d'un colorant vital (le rouge neutre) capté par les cellules vivantes.

Environ trois heures après, le colorant en excès est éliminé par lavage et le colorant capté est extrait par un volume déterminé d'une solution d'acide acétique et d'éthanol.

La cytotoxicité est déterminée par rapport à un témoin de croissance absolue où le milieu de culture 2x a été dilué au demi avec de l'eau pour préparation injectable (i.e. de l'eau déminéralisée et bidistillée) dans lequel les cellules sont laissées cinq jours pour arriver à subconfluence, c'est-à-dire à forte densité.

Un témoin positif est systématiquement effectué à l'aide d'une substance toxique de référence ($HgCl_2$, ...). La préparation du témoin positif implique de diluer au demi un milieu de culture 2x avec de l'eau pour préparation injectable, d'additionner 6 μg/ml de $HgCl_2$ et de laisser les cellules cinq jours dans ce milieu dilué et toxique.

La cytotoxicité relative pour chaque éluat est exprimée par différence avec le témoin de croissance absolue en pourcentage d'inhibition de croissance cellulaire (% ICG).

La coloration de la solution d'acide acétique et d'éthanol est fonction de la concentration en cellules vivantes et est mesurée à l'aide d'un lecteur de plaques dans l'UV/visible, selon la procédure suivante :

$$ICG\ (\%) = 100\ (D-d)/D$$

où

D représente la densité optique du témoin de croissance absolue,
d représente la densité optique de l'échantillon.

En outre, les résultats reportés dans le tableau ci-après correspondent à la moyenne de trois échantillons testés.

Dans le tableau ci-après sont reportés les résultats des mesures de cytotoxicité des PUR des exemples 1a, 1b, 2a et 2b.

| **EXEMPLE N°** | **% ICG de l'éluat (37°C/48h - 12cm$^2$/1ml)** | |
|---|---|---|
| | Eluat dilué au 1/2 | Eluat dilué au 1/4 |
| 1a | 67 | 8 |
| 1b | 26 | 10 |
| 2a | 78 | 36 |
| 2b | 25 | 9 |

Au vu de ces résultats, il apparaît nettement que les formulations de PUR des exemples 1b et 2b, incluant le composé phosphite PTP, conduisent aux valeurs les plus faibles d'inhibition de croissance cellulaire (% ICG). Conformément à l'invention, les PUR des exemples 1b et 2b sont estimés non cytotoxiques.

## Revendications

1. Masses d'empotage en polyuréthane pour échangeurs médicaux à membranes planes ou à fibres creuses, **caractérisées en ce qu'**elles sont obtenues à partir de compositions génératrices de polyuréthane comprenant :

   - au moins un polyisocyanate de préférence non aromatique, à l'état de monomère ou de prépolymère ;
   - au moins un polyol, à l'état de monomère ou de prépolymère ;
   - au moins un composé organique comprenant une ou plusieurs fonctions phosphites, à raison d'au moins 1% en poids rapporté au poids total du ou des polyols, ce composé organique étant en outre non aromatique et

porteur d'au moins un groupement hydroxy libre et capable de réagir avec un groupement isocyanato ; et

- le cas échéant, au moins un catalyseur de la réaction de polymérisation d'un polyisocyanate et d'un polyol.

2. Masses d'empotage selon la revendication 1, **caractérisées en ce que** le composé organique comprenant au moins une fonction phosphite a la formule générale (I) :

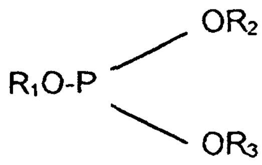

dans laquelle :

- $R_1$, $R_2$ et $R_3$ sont identiques ou différents et représentent un radical alkyle, linéaire ou ramifié, acyclique ou cyclique, mais sont exempts de groupements aromatiques ;
- au moins un radical $R_1$, $R_2$ ou $R_3$ comprend au moins un groupement hydroxy(-OH) ;

3. Masses d'empotage selon la revendication 2, **caractérisées en ce que** le ou les groupements hydroxy présents dans le ou les radicaux $R_1$, $R_2$ et $R_3$ sont primaires ou secondaires.

4. Masses d'empotage selon la revendication 2, **caractérisées en ce que** au moins un radical parmi $R_1$, $R_2$ et $R_3$ comprend au moins deux fonctions phosphites.

5. Masses d'empotage selon la revendication 2, **caractérisées en ce que** le point de fusion du composé organique est inférieur ou égal à 40°C.

6. Masses d'empotage selon la revendication 5, **caractérisées en ce que** le composé organique est liquide à température ambiante.

7. Masses d'empotage selon la revendication 6, **caractérisées en ce que** le composé organique est choisi parmi le groupe contenant l'heptakis (dipropylèneglycol)triphosphite et le tris(dipropylèneglycol)phosphite.

8. Masses d'empotage selon l'une quelconque des revendications précédentes, **caractérisées en ce que** la quantité de composé organique n'excède pas 10 % en poids rapporté au poids total du ou des polyols.

9. Masses d'empotage selon la revendication 8, **caractérisées en ce que** la quantité de composé organique est comprise entre 1,5 et 8 % en poids rapporté au poids total des polyols.

10. Procédé permettant de réduire la cytotoxicité des articles médicaux en polyuréthane susceptible d'apparaître après stérilisation ou désinfection par un procédé oxydant, **caractérisé en ce que** l'on prépare une composition génératrice de polyuréthane à partir d'au moins un polyisocyanate, de préférence non aromatique, à l'état de monomère ou de prépolymère, d'au moins un polyol, d'au moins un composé organique contenant une ou plusieurs fonctions phosphites, à raison d'au moins 1 % en poids rapporté au poids total du ou des polyols, ce composé organique étant en outre non aromatique et porteur d'au moins un groupement hydroxy libre et capable de réagir avec un groupement isocyanato et, le cas échéant, d'au moins un catalyseur de la réaction de polymérisation d'un polyisocyanate et d'un polyol.

11. Polyols utiles à la préparation des masses d'empotage en polyuréthane définies dans l'une des revendications 1 à 9, **caractérisés en ce qu'**ils sont constitués de la combinaison suivante :

- environ 45 % en poids d'un polyol à base d'esters de polyol et polyéther polyols ;
- environ 25 % d'huile de ricin ;
- environ 30 % de polycaprolactone polyol.

12. Polyols utiles à la préparation des masses d'empotage en polyuréthane définies dans l'une des revendications 1

à 9, **caractérisés en ce qu'**ils sont constitués de la combinaison suivante :

- environ 70 % en poids d'un ester de dimère diacide et d'éthylène glycol ;
- environ 15 % d'huile de ricin ;
- environ 15 % de N,N,N',N'-tétrakis (2-hydroxy-propyl)éthylène diamine.

**Patentansprüche**

1. Einbettmassen aus Polyurethan für medizinische Austauscher mit Flachmembranen oder Hohlfasermembranen, **dadurch gekennzeichnet, dass** sie aus erzeugenden Polyurethanzusammensetzungen erhalten wurden, welche umfassen:

    - wenigstens ein Polyisocyanat, bevorzugt nicht aromatisch, im monomeren Zustand oder Prepolymerenzustand;
    - wenigstens ein Polyol im monomeren Zustand oder Prepolymerenzustand;
    - wenigstens eine organische Verbindung, die ein oder mehrere Phosphitfunktionen umfasst, in einer Menge von wenigstens 1 Gew.-%, bezogen auf das Gesamtgewicht des oder der Polyols/Polyole, wobei diese organische Verbindung außerdem nicht aromatisch ist und wenigstens eine freie Hydroxygruppe trägt und in der Lage ist, mit einer Isocyanatogruppe zu reagieren; und
    - gegebenenfalls wenigstens einen Katalysator für die Polymerisationsreaktion von einem Polyisocyanat und von einem Polyol.

2. Einbettmassen nach Anspruch 1, **dadurch gekennzeichnet, dass** die organische Verbindung wenigstens eine Phosphitfunktion der allgemeinen Formel (I) umfasst:

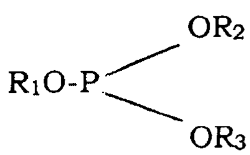

worin:

    - $R_1$, $R_2$ und $R_3$ gleich oder verschieden sind und einen geraden oder verzweigten, acyclischen oder cyclischen Alkylrest darstellen, jedoch frei von aromatischen Gruppen sind;
    - wenigstens einer der Reste $R_1$, $R_2$ oder $R_3$ wenigstens eine Hydroxygruppe (OH) umfasst.

3. Einbettmassen nach Anspruch 2, **dadurch gekennzeichnet, dass** die in dem oder den Rest/en $R_1$, $R_2$ oder $R_3$ vorhandene/n Hydroxygruppe/n primär oder sekundär ist/ sind.

4. Einbettmassen nach Anspruch 2, **dadurch gekennzeichnet, dass** wenigstens einer der Reste $R_1$, $R_2$ oder $R_3$ wenigstens zwei Phosphitfunktionen umfasst.

5. Einbettmassen nach Anspruch 2, **dadurch gekennzeichnet, dass** der Schmelzpunkt der organischen Verbindung unter oder gleich 40°C ist.

6. Einbettmassen nach Anspruch 5, **dadurch gekennzeichnet, dass** die organische Verbindung bei Umgebungstemperatur flüssig ist.

7. Einbettmassen nach Anspruch 6, **dadurch gekennzeichnet, dass** die organische Verbindung aus der Gruppe ausgewählt ist, die Heptakis(dipropylenglycol)triphosphit und Tris(dipropylenglycol)phosphit enthält.

8. Einbettmassen nach irgendeinem der vorhergehenden Ansprüche, **dadurch gekennzeichnet, dass** die Menge der organischen Verbindung 10 Gew.-%, bezogen auf das Gesamtgewicht des oder der Polyols/e, nicht übersteigt.

9. Einbettmassen nach Anspruch 8, **dadurch gekennzeichnet, dass** die Menge der organischen Verbindung zwischen 1,5 und 8 Gew.-%, bezogen auf das Gesamtgewicht der Polyole, liegt.

**10.** Verfahren, welches es erlaubt, die Cytotoxizität von medizinischen Artikeln aus Polyurethan, die nach Sterilisation oder Desinfektion durch ein oxidierendes Verfahren auftreten könnte, zu reduzieren, **dadurch gekennzeichnet, dass** man eine erzeugende Polyurethanzusammensetzung herstellt aus wenigstens einem Polisocyanat, bevorzugt nicht aromatisch, im monomeren Zustand oder Prepolymerenzustand; wenigstens einem Polyol; wenigstens einer organischen Verbindung, die ein oder mehrere Phosphitfunktionen in einer Menge von wenigstens 1 Gew.-%, bezogen auf das Gesamtgewicht des oder der Polyols/Polyole, hat, wobei diese organische Verbindung außerdem nicht aromatisch ist und wenigstens eine freie Hydroxygruppe trägt und in der Lage ist, mit einer Isocyanatogruppe zu reagieren; und gegebenenfalls wenigstens einen Katalysator für die Polymerisationsreaktion von Polyisocyanat und von einem Polyol.

**11.** Polyole, welche für die Herstellung von Einbettmassen aus Polyurethan verwendbar sind, wie in einem der Ansprüche 1 bis 9 definiert, **dadurch gekennzeichnet, dass** sie durch die folgende Kombination gebildet werden:

- ungefähr 45 Gew.-% eines Polyols auf Basis von Polyolestern und Polyetherpolyolen;
- ungefähr 25 Gew.-% Rizinusöl;
- ungefähr 30 Gew.-% Polycaprolactonpolyol.

**12.** Polyole, welche für die Herstellung von Einbettmassen aus Polyurethan verwendbar sind, wie in einem der Ansprüche 1 bis 9 definiert,
**dadurch gekennzeichnet, dass** sie durch die folgende Kombination gebildet werden:

- ungefähr 70 Gew.-% eines Esters von Disäuredimeren und von Ethylenglycol;
- ungefähr 15 Gew.-% Rizinusöl;
- ungefähr 15 Gew.-% N,N,N',N'-Tetrakis(2-hydroxypropyl)ethylendiamin.

## Claims

**1.** Polyurethane potting bodies for flat-membrane or hollow-fibre medical exchangers, **characterized in that** they are obtained from polyurethane-generating compositions comprising:

- at least one polyisocyanate, preferably a non-aromatic polyisocyanate, in the monomer or prepolymer state;
- at least one polyol in the monomer or prepolymer state;
- and at least one organic compound comprising one or more phosphite functional groups, in the proportion of at least 1% by weight with respect to the total weight of the polyol or polyols, this organic compound being, in addition, non-aromatic and the carrier of at least one free hydroxyl group and capable of reacting with an isocyanato group; and
- if appropriate, at least one catalyst of the polymerization reaction of a polyisocyanate and of a polyol.

**2.** Potting bodies according to Claim 1, **characterized in that** the organic compound comprising one or more phosphite functional groups has the general formula (I):

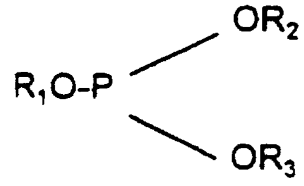

in which:

- $R_1$, $R_2$ and $R_3$ are identical or different and represent a linear or branched, acyclic or cyclic alkyl radical but are devoid of aromatic groups;
- at least one radical $R_1$, $R_2$ or $R_3$ comprises at least one hydroxyl group (-OH).

3. Potting bodies according to Claim 2, **characterized in that** the hydroxyl group or groups present in the radical or radicals $R_1$, $R_2$ and $R_3$ are primary or secondary.

4. Potting bodies according to Claim 2, **characterized in that** at least one radical chosen from the group of $R_1$, $R_2$ and $R_3$ comprises at least two phosphite functional groups.

5. Potting bodies according to Claim 2, **characterized in that** the melting point of the organic compound is less than or equal to 40°C.

6. Potting bodies according to Claim 5, **characterized in that** the organic compound is liquid at room temperature.

7. Potting bodies according to Claim 6, **characterized in that** the organic compound is chosen from the group comprising heptakis(dipropylene glycol) triphosphite and tris(dipropylene glycol) phosphite.

8. Potting bodies according to any one of the preceding claims, **characterized in that** the amount of organic compound does not exceed 10% by weight with respect to the total weight of the polyol or polyols.

9. Potting bodies according to Claim 8, **characterized in that** the amount of organic compound is between 1.5 and 8% by weight with respect to the total weight of the polyols.

10. Process which makes it possible to reduce the cytotoxicity of polyurethane medical items liable to appear after sterilization or disinfection by an oxidizing process, **characterized in that** a polyurethane-generating composition is prepared from at least one polyisocyanate, preferably a non-aromatic polyisocyanate, in the monomer or prepolymer state, from at least one polyol, from at least one organic compound comprising one or more phosphite functional groups, in the proportion of at least 1% by weight with respect to the total weight of the polyol or polyols, this organic compound being, in addition, non-aromatic and the carrier of at least one free hydroxyl group capable of reacting with an isocyanato group, and, if appropriate, from at least one catalyst of the polymerization reaction of a polyisocyanate and of a polyol.

11. Polyols of use in the preparation of the polyurethane potting bodies defined in one of Claims 1 to 9, **characterized in that** they are composed of the following combination:

- approximately 45% by weight of a polyol based on polyol ester and on polyol ether;
- approximately 25% of castor oil;
- approximately 30% of polycaprolactone polyol.

12. Polyols of use in the preparation of the polyurethane potting bodies defined in one of Claims 1 to 9, **characterized in that** they are composed of the following combination:

- approximately 70% by weight of an ethylene glycol dimerate;
- approximately 15% of castor oil;
- approximately 15% of N,N,N'N'-tetrakis(2-hydroxypropyl)ethylenediamine.